# NEW EUROPEAN PATENT SPECIFICATION

(11) **EP 0 360 285 B2**
(45) Date of publication and mention of the opposition decision: **10.03.1999**
(45) Mention of the grant of the patent: 03.01.1996
(21) Application number: 89117573.9
(22) Date of filing: 22.09.1989
(51) Int. Cl.: A61F 13/15

(54) **Absorbent body having hydrophobic insert**
Saugfähiger Körper mit hydrophober Zwischenschicht
Corps absorbant avec une pièce rapportée hydrophobe

(30) Priority: 22.09.1988 US 247479
(43) Date of publication of application: 28.03.1990
(73) Proprietor: McNEIL-PPC, INC., Milltown New Jersey 08850 (US)
(72) Inventor: Dabroski, Winifred C., East Brunswick, N.J. 08816 (US)
(74) Representative: Groening, Hans Wilhelm, Dipl.-Ing.

(56) References cited:
- EP-A- 134 086
- EP-A- 211 376
- DE-A- 3 718 076
- GB-A- 807 768
- GB-A- 1 277 216
- US-A- 2 965 102
- US-A- 3 375 827
- US-A- 4 072 151
- US-A- 4 195 634
- US-A- 4 354 901

## Description

### Background Of The Invention

The invention relates to an absorbent article having oppositely disposed ends and longitudinal edges.

More particularly, this invention relates to an absorbent pad for use in such products as sanitary napkins, panty liners, diapers and the like.

In general, such products comprise one or more layers of a core of hydrophilic material such as wood pulp, rayon tissue or the like. The hydrophilic material, generally fibrous in form, is provided as a pad having a rectangular or oblong shape or in some cases, a shape designed to fit the anatomy of the wearer more closely. Such products may also be designed to have wings or flaps, which extend transversely from the product and serve to protect the wearer's panty from becoming stained due to the tendency for body fluid to flow over the sides of the napkin. The pad is usually provided with an enveloping cover pervious to body fluids on the side of the pad which is to be placed against the body and impervious to such fluids on the side facing away from the body. The object of such a body fluid impervious cover is, of course, to protect the clothing from staining and wetting.

In general, such products have satisfactorily performed their function of absorbing and retaining body fluids and preventing staining and wetting of the wearer's clothing. When the product is properly placed and retained by the wearer in its intended position, body fluid is directed at or near the center of the product and is distributed, by means of liquid wicking, throughout the absorbent medium. However, in a significant number of cases, the product is misplaced, either by the wearer when she initially places the product, or by her activities. Under these circumstances, body fluid will strike the pad off-center and closer to the peripheral edges of the pad. This off-center deposition of body fluid is believed to be the cause of a significant number of failures associated with the use of these products, i.e., the staining and wetting the clothing of the wearer by body fluid. Such failures are known as "side failures".

Even when the product has not been misplaced by the wearer, there are a significant number of failures at the sides of the product. Flow through the absorbent medium after deposition of body fluid is both lateral and longitudinal. Because the distance between the sides of the product is relatively small, fluid can travel laterally and cause side failure quickly.

In the past, there have been efforts to direct the flow of body fluid using baffles or densified sheets.

US 2,965,102 discloses a sanitary napkin having a baffle disposed approximately midway in a main pad body between two center plies of crepe tissue forming a pad body. The napkin includes a supplementary pad body on one side for normal use whilst the opposite side provides some absorbency in case the pad is intentionally or improperly positioned with the pad body diposed away from the body of the wearer. In the latter respect, the baffle does not extend the full length of the main pad, so that fluid can enter the main pad body at the ends. The baffle sheet of fluid-repellent material can also be disposed between the main and supplementary main pad bodies and is generally coextensive in area with the latter. The use of semi-permeable material is preferred to allow fluid to pass through into the main pad body under pressure.

EP-A-0 211 376 discloses a panty liner with a flow retarding layer which is based on the object to provide a panty liner with a means for minimizing strike through under unusually heavy liquid deposition without sacrificing the desirable attributes of breathability. It is provided a core contained between a cover and a backing. The core comprises a flow retarding means sandwiched between a body side absorbent element and a garment side absorbent element. The function of the flow retarding means is to preclude a sudden large surge of body fluid deposited on a relatively small area of the product from passing unhindered through the body side absorbent element into the garment side absorbent element, overburdening the barrier system, and thereby staining the wearer's undergarment. Due to the presence of the flow retarding means, such a sudden surge of fluid is retarded from flow from the body side to the garment side and is instead directed laterally and dispersed to the body side absorbent element. On the other hand the flow retarding means must be capable of allowing for fluid flow therethrough, primarily so that fluid, in the form of vapour, may pass through the product and hence the product can quickly dry while being worn. Additionally, should the body side absorbent element become heavily loaded with fluid, then it is important to have the capability of transporting such fluid through the flow retarding means and retaining such fluid in the garment side absorbent element.

As to achieve these functions of the flow retarding means, it can be provided a layer comprising hydrophobic fibers.

Suitable material for use as a flow retarding means is one or more plies of a tissue paper which is treated by applying thereto a chemical agent which gives the tissue the required resistance to flow and hydrophobicity. Cellulose fibers for a ply of untreated tissue are preferred. As a flow retarding layer can also be used a web comprised of synthetic hydrophobic polymer fibers.

US 4,195,534 discloses a sanitary napkin with resilience stiffening means as to exhibit dimensional stability during use, resisting compressive forces exerted along the side portions of the napkin, remaining its original position on the undergarment to which it is attached, comfortable when worn and resistant to fluid leakage and thereby preventing side staining. The sanitary napkin comprises an absorbent core, a liquid pervious material surrounding the core, a sheet of a liquid impervious material forming a moisture barrier at the bottom portion of the napkin and stiffener means positioned between the core and the liquid impervious material consisting of a material having a high enough level of resiliency and stiffness to resist side compression to a sufficient degree to aid in retaining the adhesive means in place on the undergarment when compressive forces are applied to the napkin during use. A pulp core is formed of two elements of a pulp fluff material and three separate layers of a carrier tissue paper. The tissue paper layers serve a dual purpose, i.e. they facilitate manufacturing the napkin and help to spread the menstrual fluids evenly so as to provide for maximum absorption of these fluids into the napkin. Examples of material for the stiffener means include spun bonded polyester materials and forms formed of polyurethane, cellulose, polyethylene latex, heavy films formed of polyethylene and laminates. Furthermore, the width of the stiffener means is less than the width of said napkin.

US 4,354,901 discloses flexible absorbent boards comprising an absorbent material being provided for body fluid absorbent products which material has relatively high tensile strength, is extremely flexible and may be incorporated into absorbent products without substantial loss in user's comfort. A sheet of flexible hydrocolloidal particle containing board is placed between a barrier sheet and a pad. A board is positioned centrally within a pad. This arrangement is provided for an improvement in the manufacture of absorbent bodies specifically directed toward increasing the integrity of the bodies without sacrificing flexibility, particularly when introducing chemically modified absorbent materials into the absorbent body.

EP-A-0 134 086 is related to a shaped sanitary napkin with flaps. The napkin has an absorbent core and two flaps extending outwardly from the absorbent means. Each flap is associated with the absorbent means along a non-linear line of juncture and each flap has two axes of flexibility. In use, the absorbent means is secured in the crotch of a panty and the flaps are secured about the elastic edges of the panty so that, when the panty is worn, the absorbent means is held adjacent the body while the flaps form a wall at each side of the absorbent means and seals against the body. Both the walls and the seals serve to prevent soiling of the body and clothing by menstrual fluid. The backsheet is the liquid impervious garment facing surface of the napkin and serves also as a backsheet for flaps but can also be an independent element although the first mentioned version is preferred.

U.S. Patent No. 3,375,827 (Bletzinger et al.) describes a flow control element located on the body facing side of a main absorbent element of a sanitary napkin. Bletzinger et al. describe the flow control element as a compressed strip of absorbent material of smaller dimension in length and width than the main absorbent element. However, the flow control element rests directly on top of the absorbent core. Thus, the fluid flows radially around and through the flow control element to the core. The fluid, therefore, can flow transversely to the sides of the pad as well as longitudinally, causing side failure. GB-A-807,768 discloses an intermediate sheet having barrier stripes of paraffin.

U.S. Patent No. 3,612,054 (Matsuda) describes a sanitary napkin having three layers of absorbent material and barrier sheets of liquid repellent material interposed between the layers. Although the barrier sheets are intended to utilize the full capacity of the absorbent layers, they do not address the problem of side failure. Fluid is able to leak throughout the absorbent layers and out the sides of the napkin.

U.S. Patent No. 4,282,874 (Mesek) describes a diaper having a densified layer. The densfied layer is intended to provide a "wickability" gradient which rapidly transmits fluid away from the body-facing layer without permitting it to flow back toward the body. Mesek does not, however, address the problem of preventing leakage at the sides of an absorbent article.

Thus, it is an object of this invention to provide an absorbent article which substantially prevents the flow of body fluid to the sides of the article so as to limit or prevent side failure.

It is another object of this invention to provide an absorbent article such as a sanitary napkin which is capable of directing flow of body fluid along the longitudinal axis of the article.

Yet another object of this invention is to provide an absorbent product in which a large proportion of the absorbent capacity is utilized.

### Summary Of The Invention

The afore-mentioned objects are achieved by the features as disclosed in claim 1.

In accordance with the principles of the present invention, an absorbent product is provided which directs fluid flow along the longitudinal direction of the product and substantially limits or prevents side failure by substantially preventing the flow of fluid around the longitudinal edges of the product In the absorbent product of this invention, a fluid repellent layer substantially prevents contact between absorbent layers along the longitudinal edges of the product by separating the absorbant layers along their longitudinal edges. The fluid repellent layer permits contact between the top layer and an absorbent layer below the fluid repellent layer at the longitudinal ends of the produce. Without an outlet for the fluid at the longitudinal edges, the fluid flows along the longitudinal axis of the absorbent product toward the ends of the product, and is absorbed into one or more bottom reservoir layers.

The longitudinal edges of the first and second absorbent layers should be separated along a substantial portion of their length.

When body fluid is applied to the absorbent products of this invention, the fluid flows into the first absorbent layer and, being prevented from flowing transversely across the product, must flow toward the ends of the product along the longitudinal axis, around the ends of the fluid repellent layer and into the second absorbent layer.

The first and second and additional absorbent layers may be composed of a variety of materials, including wood cellulose fibers, wood pulp, regenerated cellulose or cotton fibers, rayon or other synthetic fibers and/or other materials known to those of skill in the art to be able to absorb and retain fluids. The absorbent layers are preferably composed of wood pulp, as it has an extremely high capacity for absorbing and retaining fluids. The absorbent layers may be rectangular or shaped in a desired manner.

The top and bottom absorbent layers may be made of materials having different degrees of absorbency and retention. For example, the bottom layer may be relatively more absorbent than the top layer. This absorbency differential would promote the transfer and flow of fluid from the top layer to the bottom layer.

The fluid repellent layer should have properties which render it repellent to fluid relative to the absorbent layers. Thus, it need not be composed only of foams or other materials which are considered by those in the art to be "hydrophobic", i.e. which does not absorb or adsorb fluid. Preferably, the fluid repellent layer is a polymeric foam. Not only is such a foam fluid repellent, but it provides resiliency to the sanitary napkin, allowing it to retain its shape and aid in maintaining good contact between the absorbent product and the user's body. For example, the fluid repellent layer may be a urethane foam, a polyethylene foam, a styrene foam, a rubber foam, an aminoether foam, or the like. More preferably, the foam is a urethane foam. The foam should be perceived to be comfortable by the wearer. It should also be somewhat resilient in order to protect the pad from being crushed or mangled during use. Preferably, the foam density should be between about 0.035 g/cm³ and about 0.320 g/cm³ The stiffness of the foam as measured on a Gurley Stiffness Tester (manufactured by W. and L.E. Gurley of Troy, N.Y. as described in U.S. Patent No. 4,354.901, issued October 19. 1982 to Kopolow) should be between about 1 and 9. More preferably, it should be between about 2 and 6.

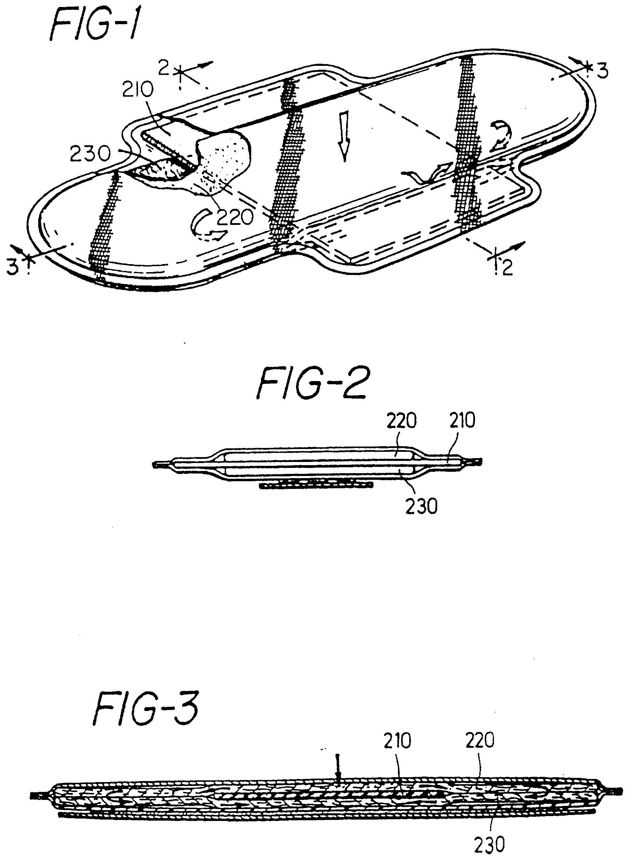

Figures 1-3 represent a preferred embodiment of a sanitary napkin according to this invention which has very short side projections, or "winglets" formed by fluid repellent layer side projections. Bottom absorbent layer 230, which may be composed of wood pulp fluff is coterminous with top absorbent layer 220 which may also be made of wood pulp fluff. Fluid repellent layer 210 is superimposed over bottom absorbent layer 230 and extends on either side beyond the width of the top and bottom absorbent layers. The side projections of fluid repellent layer 210 not only protect against side failure by preventing the contact between the absorbent layers at the side or longitudinal edges, but they provide extra protection for the wearer's undergarments. They also provide resilient edges which aid in preventing chaffing and irritation of the legs when contacting the side wings.

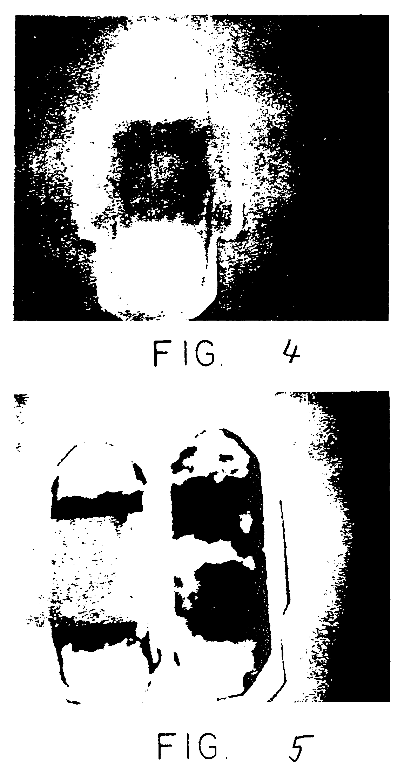

Figures 4 and 5 depict photographs of a sanitary napkin made in accordance with this invention. The fluid repellent layer of the napkin has winglet side projections. Ersatz menstrual fluid has been deposited on the napkin and the flow patterns examined. Figure 4 demonstrates that the portion of the top repellent layer which is coterminous with the fluid repellent layer is saturated with fluid. Figure 5 demonstrates that the fluid flows preferentially over the longitudinal ends of the fluid repellent layer into the bottom absorbent layer rather than over the sides, which would cause side failure.

The effectiveness of using the products of this invention is demonstrated by the following examples. Of course, these examples are not intended to limit the scope of the invention in any way, but merely serve to illustrate the products of this invention and the use thereof.

### Example 1

A sanitary napkin was constructed using an embossed polypropylene backing having a triple row of Fuller 1940W hot melt positioning adhesive (a styrene-ethylene-butadiene-styrene block copolymer resin and oil available from H. P. Fuller Co.), two compressed light weight pulp pads having a total weight of 5.0g, a fluid repellent layer made of General Foam P3800 (a urethane foam film available from General Foam Co.) approximately 76.2 mm (3 inches) long x 101.6 mm (4 inches) wide x 3.18 mm (1/8 inch) deep located on top of the compressed pulp pads, and a fluffy layer of pulp about 190.5 mm (7.5 inches) long x 66.0 mm (2.6 inches) wide on top of the foam. The product was covered with an apertured film made of coextruded polyethylene and ethylvinylacetate described in U.S. Patent No. 4,690,679 using a spray. The cover was flange sealed to the polypropylene backing. The napkin was formed with side panel projections each having a width of about 17.8 mm (0.7 inches).

### Example 2

A sanitary napkin was formed using a pulp pad having dimensions of 190.5 mm (7-1/2 inches) long x 76.2 mm (3 inches) wide. Over the pulp pad was placed a 3.18 mm (1/8 inch) thick x 76.2 mm (3 inches) long x 101.6 mm (4 inches) wide General Foam urethane pad which was to act as a fluid repellent layer. A smaller pulp pad was glued over the foam layer. A 0.054 mm (0.001 inch) thick white embossed polyethylene backing was placed under the napkin and flange sealed to a coextruded apertured film cover using cover spray adhesive.

### Example 3

A sanitary napkin was made using a light weight pulp pad compressed in the center and a urethane foam pad 76.2 mm (3 inches) wide x 44.45 mm (1-3/4 inches) long x 9.53 mm (3/8 inches) thick which was laid into the compressed area of the pulp pad. A smaller pulp pad was placed over the foam. The foam was glued to both pulp pads. The pad was overwrapped with a fibrous dry cover using cover spray.

The following Examples are illustrative of in vivo menstrual use of the products of this invention.

### Example A

Menstruating women were given a number of sanitary napkins for alternative testing. Twenty women each received six sanitary napkins made in accordance with Example 1 and six Regular ALWAYS™ brand Regular Maxipad sanitary napkins commercially available from The Procter & Gamble Company. The pads were numbered consecutively and the women instructed to use the pads in consecutive numerical order. The pads were to be worn for six hours or until failure, whichever was first. Failure was defined as leakage of menstrual fluid from the side of a napkin, from the ends of a napkin or from a combination of the sides and the ends. Of the Example 1 sanitary napkins, six of the forty-eight napkins used exhibited a failure, or 12.5%. Fourteen of the fifty-one ALWAYS™ maxipads used resulted in a failure, or 27.5%.

### Example B

An alternate use test was conducted in accordance with the procedure outlined in Example A, with the exception that the sanitary napkins of Example 2 were used in alternation with ALWAYS™ Regular Maxipads available from Procter & Gamble. The use of the napkins of Example 2 resulted in eight failures out of fifty-four napkins, or 14.8% failures. The use of ALWAYS™ Regular Maxipads, in comparison with the use of the napkins made according to this invention, resulted in twenty-four failures out of fifty-five napkins used, or 43.64% failures. This indicates that the napkins made in accordance with this invention are more effective in preventing failures than the commercially available ALWAYS™ Regular Maxipad product.

### Example C

An alternate use test was conducted in accordance with the procedure outlined in Example A, with the exception that the sanitary napkins of Example 3 were used in place of the napkins described in Example A with ALWAYS™ Brand Regular "New-Longer" Maxipads. Use of the napkins of Example 3 resulted in ten failures out of sixty-seven napkins used, or 14.93% failures. The alternatively-used napkins, ALWAYS™ Brand Longer napkins resulted in thirty-six failures out of sixty-eight napkins used, or 52.94% failures. This comparison demonstrates the unexpectedly superior performance of the products of Applicant's invention in comparison with napkins lacking a fluid repellent layer in accordance with this invention.

## Claims

1. An absorbent article having oppositely disposed ends and longitudinal edges, comprising:
(a) a fluid permeable cover with a body contacting surface;
(b) a first absorbent layer (220) subjacent to said cover having oppositely disposed longitudinal edges, and oppositely disposed transverse edges, a body facing side and a garment facing side;
(c) a fluid repellent layer (210) underlying said first absorbent layer (220) having oppositely disposed longitudinal edges and oppositely disposed transverse edges;
(d) a second absorbent element (230) having oppositely disposed longitudinal edges and oppositely disposed transverse edges, underlying said fluid repellent layer (210);
wherein
(e) the distance between said longitudinal edges of said fluid repellent layer (210) being greater than that of the first absorbent element (220) and the distance between said transverse edges of the fluid repellent layer being less than that of said transverse edges of the first absorbent element 220);
(f) the distance between said transverse edges of said second absorbent element (230) being greater than that of said transverse edges of the fluid repellent layer (210);
whereby said first and second absorbent layer (220, 230) are substantially separated a distance along their longitudinal edges such that fluid striking said first absorbent layer (220) is substantially prevented from flowing around the longitudinal edges and substantially flows along the longitudinal direction of the absorbent article into said second absorbent element (230).

2. The absorbent article according to claim 1, wherein the distance between the longitudinal side edges of said second absorbent layer is greater than that of the first absorbent layer.

3. The absorbent article according to claim 1, wherein said fluid repellent layer (210) comprises a material selected from the group consisting of fibrous materials and polymeric foams.

4. The absorbent article according to claim 3, wherein said fluid repellent layer (210) comprises fibrous materials.

5. The absorbent article according to claim 1, wherein said fluid repellent layer (210) comprises a foam having a Gurley Stiffness of between about 1 and about 9.

6. The absorbent article according to claim 5, wherein said Gurley Stiffness is between about 2 and about 6.

7. The absorbent article according to claim 5, wherein the density of said foam is between about 0.035 g/cm³ and 0.320 g/cm³.

## Patentansprüche

1. Absorbierender Artikel mit gegenüberliegend angeordneten Enden und Längsrändern, umfassend:
(a) eine flüssigkeitsdurchlässige Abdeckung mit einer den Körper berührenden Oberseite;
(b) eine erste Absorptionsschicht (220) unter der Abdeckung mit gegenüberliegend angeordneten Längsrändern und gegenüberliegend angeordneten, quer verlaufenden Rändern, einer dem Körper zugewandten Seite und einer der Kleidung zugewandten Seite;
(c) eine flüssigkeitsabweisende Schicht (210) unter der ersten Absorptionsschicht mit gegenüberliegend angeordneten Längsrändern und gegenüberliegend angeordneten, quer verlaufenden Rändern;
(d) ein zweites Absorptionselement (230) mit gegenüberliegend angeordneten Längsrändern und gegenüberliegend angeordneten, quer verlaufenden Rändern, die unter der flüssigkeitsabweisenden Schicht (210) liegen;
wobei
(e) der Abstand zwischen den Längsrändern der flüssigkeitsabweisenden Schicht (210) größer ist als derjenige des ersten Absorptionselements (220) und der Abstand zwischen den quer verlaufenden Rändern der flüssigkeitsabweisenden Schicht kleiner ist als derjenige der quer verlaufenden Ränder des ersten absorbierenden Elements (220);
(f) der Abstand zwischen den quer verlaufenden Rändern des zweiten Absorptionselements (230) größer ist als derjenige der quer verlaufenden Ränder der flüssigkeitsabweisenden Schicht (210);
wobei die erste und zweite Absorptionsschicht (220, 230) entlang ihrer Längsränder im wesentlichen in einem Abstand von einander derart getrennt sind, daß die auf die erste Absorptionsschicht (220) auftreffende Flüssigkeit im wesentlichen an einem Herumfließen um die gehindert wird und im wesentlichen in Längsrichtung des absorbierenden Artikels in das zweite Absorptionselement (230) fließt.

2. Absorbierender Artikel nach Anspruch 1, bei dem der Abstand zwischen den längsseitigen Rändern der zweiten Absorptionsschicht größer ist als derjenige der ersten Absorptionsschicht.

3. Absorbierender Artikel nach Anspruch 1, bei dem die flüssigkeitsabweisende Schicht (210) ein Material umfaßt, das ausgewählt ist aus der Gruppe bestehend aus faserartigen Materialien und polymeren Schäumen.

4. Absorbierender Artikel nach Anspruch 3, bei dem die flüssigkeitsabweisende Schicht faserartige Materialien umfaßt.

5. Absorbierender Artikel nach Anspruch 1, bei dem die flüssigkeitsabweisende Schicht (210) einen Schaum mit einer Gurley-Steifheit von zwischen etwa 1 und etwa 9 umfaßt.

6. Absorbierender Artikel nach Anspruch 5, bei dem die Gurley-Steifheit zwischen etwa 2 und etwa 6 beträgt.

7. Absorbierender Artikel nach Anspruch 5, bei dem die Dichte des Schaums zwischen etwa 0,035 und 0,320 g/cm² beträgt.

## Revendications

1. Article absorbant présentant des extrémités et des bords longitudinaux disposés de façon opposée comprenant :
(a) une enveloppe perméable aux fluides présentant une surface en contact avec le corps ;
(b) une première couche absorbante (220) sous-jacente à ladite enveloppe présentant des bords longitudinaux disposés de façon opposée et des bords transversaux disposés de façon opposée, un côté faisant face au corps et un côté faisant face au sous-vêtement ;
(c) une couche imperméable aux fluides (210) posée sous ladite première couche absorbante (220) présentant des bords longitudinaux disposés de façon opposée et des bords transversaux disposés de façon opposée ;
(d) un second élément absorbant (230) présentant des bords longitudinaux disposés de façon opposée et des bords transversaux disposés de façon opposée, placé sous ladite couche imperméable aux fluides (210),
dans lequel
(e) la distance entre lesdits bords longitudinaux de la dite couche imperméable aux fluides (210) est supérieure à celle du premier élément absorbant (220) et la distance entre lesdits bords transversaux de la couche imperméable aux fluides est inférieure à celle desdits bords transversaux du premier élément absorbant (220) ;
(f) la distance entre lesdits bords transversaux dudit second élément absorbant (230) est supérieure à celle desdits bords transversaux de la couche imperméable aux fluides (210),
lesdites première et seconde couches absorbantes (220,230) étant sensiblement séparées d'une certaine distance le long de leurs bords longitudinaux de sorte que le fluide touchant ladite première couche absorbante (220) ne peut sensiblement pas s'écouler autour des bords longitudinaux et s'écoule sensiblement le long de la direction longitudinale de l'article absorbant dans ledit second élément absorbant (230).

2. Article absorbant selon la revendication 1, dans lequel la distance entre les bords longitudinaux de ladite seconde couche absorbante est supérieure à celle de la première couche absorbante.

3. Article absorbant selon la revendication 1, dans lequel ladite couche imperméable aux fluides (210) comprend une matière choisie parmi les matières fibreuses et les mousses polymères.

4. Article absorbant selon la revendication 3, dans lequel ladite couche imperméable aux fluides (210) comprend des matières fibreuses.

5. Article absorbant selon la revendication 1, dans lequel ladite couche imperméable aux fluides (210) comprend une mousse ayant une rigidité Gurley entre 1 et 9 environ.

6. Article absorbant selon la revendication 5, dans lequel ladite rigidité Gurley est entre 2 et 6 environ.

7. Article absorbant selon la revendication 5, dans lequel la densité de ladite mousse est entre 0,035 et 0,320 g/cm³.
